# EUROPEAN PATENT APPLICATION

(11) **EP 1 728 473 A1**
(43) Date of publication of application: **06.12.2006**
(21) Application number: 06011496.4
(22) Date of filing: 02.06.2006
(51) Int. Cl.: A61B 17/064

(54) **Expandable backspan staple**

(30) Priority: 02.06.2005 US 686780 P
(71) Applicant: Tyco Healthcare Group LP, Norwalk, Connecticut 06856 (US)
(72) Inventor: Viola, Frank J, Sandy Hook, CT 06482 (US); Blier, Kenneth, Meriden, CT 06450 (US); Hadba, Ahmad R., Wallingford, CT 06492 (US); Heinrich, Russell, Madison, CT 06443 (US)
(74) Representative: HOFFMANN EITLE

(57) **Abstract**

A staple (10) having an expandable backspan (12) and a pair of spaced legs (14,16) is described. The expandable backspan is configured to expand or deform to accommodate tissues of varying thicknesses. In one embodiment, the backspan is non-linear and defines a recess. In another embodiment, the backspan includes a deformable pad or spacer. The amount of deformation of the backspan is proportional to the thickness of the tissue, i.e., the greater the thickness of tissue, the greater the deformation of the backspan.

## Description

### CROSS-REFERENCE TO RELATED APPLICATIONS

The present application claims the benefit of and priority to U.S. Provisional Patent Application No. 60/686,780, filed on June 2,2005, the entire contents of which are incorporated herein by reference.

### BACKGROUND

### 1. Technical Field

The present disclosure relates to surgical fasteners. More particularly, the present disclosure relates to surgical staples for use with surgical stapling instruments for joining tissue of varying thicknesses.

### 2. Background-Of Related Art

Surgical staples and stapling instruments are well known in the surgical arts and have become critical to many life saving surgical procedures. The use of stapling instruments for applying staples to join tissue or tissue segments in a fast and efficient manner has obviated the time consuming step of manual suturing of tissue or tissue segments in a variety of surgical procedures, e.g., anastomoses procedures. The reduced time required to perform these surgical procedures using surgical stapling instruments has resulted in reduced trauma and risk to patients.

Typically, a surgical staple includes a backspan and a pair of spaced legs. The legs are driven through tissue and into an anvil to deform the staple into a desired configuration, e.g., B-staple, to effect hemostasis of tissue or tissue segments. One problem associated with current surgical staples is that a deformed staple of a given size is particularly suited to effect hemostasis of tissue of a given thickness range. As such, a surgeon must choose the appropriate staple size for a given tissue thickness range to ensure effective hemostasis of tissue.

Accordingly, it would be desirable to provide a surgical staple that can accommodate a greater range of tissue thicknesses, thereby providing the surgeon greater flexibility when performing surgery.

### SUMMARY

In accordance with the present disclosure, a staple is provided which includes a non-linear expandable backspan, a first leg having a first end extending from one end of the expandable backspan and a second end, and a second leg having a first end extending from the other end of the expandable backspan and a second end. The non-linear expandable backspan includes a central portion which extends towards the second ends of the first and second legs and is deformable in a direction away from the second ends of the first and second legs. The backspan is configured to deform upon application to tissues of varying thicknesses, wherein the amount of deformation of the backspan is proportional to the thickness of tissue being stapled.

In one embodiment, the second ends of the first and second legs are configured to penetrate tissue. In one embodiment, the backspan has a concave or recessed configuration. The recessed or concave configuration can be u-shaped, trapezoidal, rectangular or any other configuration suitable to achieve the stated objectives.

In one embodiment, either or both of the backspan and first and second legs have a circular cross-section. Alternately, other cross-sectional configurations arc envisioned.

In yet another embodiment, the backspan can include a compressible pad or spacer. The pad can be positioned to engage tissue upon application of the staple to tissue to maintain approximation of tissue or tissue segments of varying thickness. In one embodiment, the compressible pad includes a polymer, a fluid filled bag or sponge. Alternately, other compressible materials can be used.

### BRIEF DESCRIPTION OF THE DRAWINGS

Various embodiments of the presently disclosed expandable backspan staple arc disclosed herein with reference to the drawings, wherein:
FIG. 1 is a perspective view of one embodiment of the presently disclosed expandable backspan staple;
FIG. 1A is a side view of another embodiment of the presently disclosed expandable backspan staple;
FIG. 1B is a side view of yet another embodiment of the presently disclosed expandable backspan staple;
FIG. 1C is a top view of yet another embodiment of the presently disclosed expandable backspan staple;
FIG. 2 is a cross-sectional view taken along section lines 2-2 of FIG. 1;
FIG. 3 is a cross-sectional view taken along section lines 3-3 of FIG. 1;
FIG. 4 is a side view of the expandable backspan staple shown in FIG. 1 positioned through tissue of minimal thickness;
FIG. 5 is a side view of the expandable backspan staple shown in FIG. 1 positioned through tissue of moderate thickness;
FIG. 6 is a side view of the expandable backspan staple shown in FIG. 1 positioned through tissue of greater thickness;
FIG. 7 is a side perspective view of another embodiment of the presently disclosed expandable backspan staple;
FIG. 8 is a side view of the expandable backspan staple shown in FIG. 7 positioned through tissue of moderate thickness; and
FIG. 9 is a side view of the expandable backspan staple shown in FIG. 7 positioned through tissue of greater thickness.

### DETAILED DESCRIPTION OF EMBODIMENTS

Embodiments of the presently disclosed expandable backspan staple will now be described in detail with reference to the drawings wherein like numerals designate identical or corresponding elements in each of the several views.

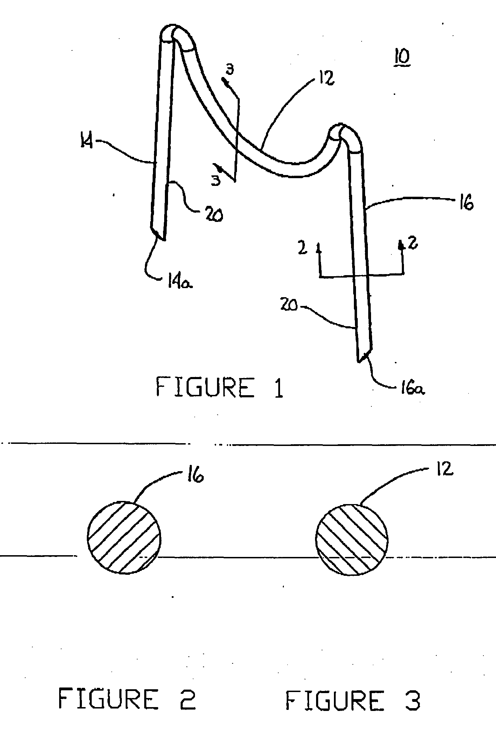

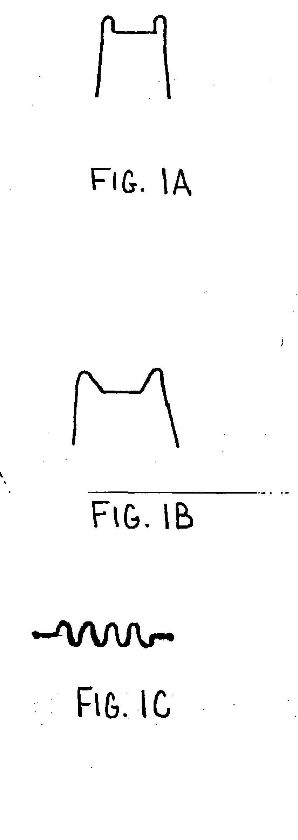

Referring to FIG. 1, the presently disclosed expandable backspan staple shown generally as 10 includes a backspan 12, a first leg 14 extending outwardly from one end of backspan 12 and a second leg 16 extending outwardly from the other end of backspan 12. Each of first and second legs includes a tissue penetrating tip 14a and 16a. Backspan 12 defines a curve or recess to provide the staple backspan with a concave profile. A central portion 12a of backspan 12 is curved or recessed such that central portion 12a extends towards the penetrating tips of staple legs 14 and 16. Alternately, the backspan of the staple, as it extends from first leg 14 to second leg 16, need not extend in a straight line when viewed from above the backspan. For example, the top profile of the backspan may extend from first leg 14 to second leg 16 in a serpentine pattem. See FIC. 1C. It is envisioned that the backspan configuration can be formed having any desired radius of curvature to suit a particular need, surgical procedure, or range of tissue thicknesses (as will be discussed below). It is also envisioned that the profile of the staple backspan need not be circular but rather may have other recessed or concave configurations, e.g., U-shaped, trapezoidal (FIG. 1B), rectangular (FIG. 1A), etc.

As illustrated in FIG. 1, penetrating tips 14a and 16a of legs 14 and 16 of staple 10 can be formed with tapered ends to facilitate penetration of tissue. Tissue penetrating tips 14a and 16a can be tapered as shown with an interior wall 20 of the staple defining an edge 22. Alternately, tissue penetrating tips 14a and 16a of the staple legs 14 and 16 need not be tapered, can be tapered in a different direction, or can define a conical or flat surface.

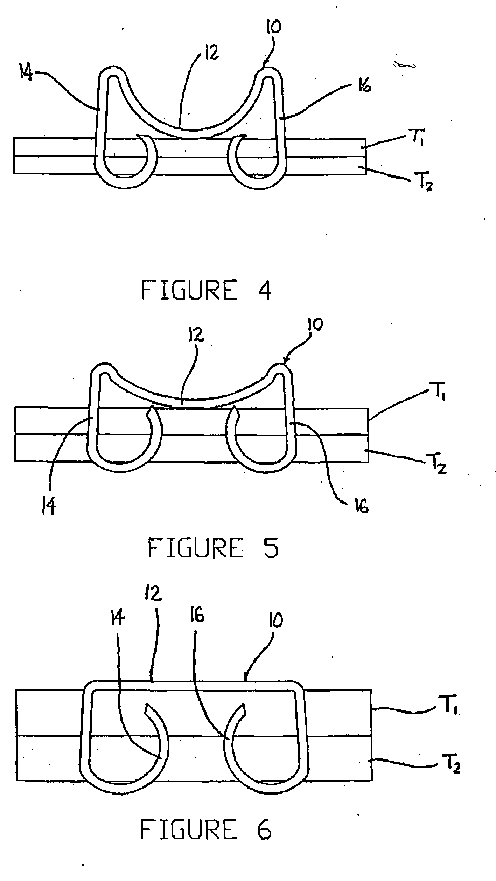

As illustrated in FIGS. 2 and 3, staple 10 can have a circular cross-section throughout its length. It is envisioned that staple 10 may have a variety of different cross-sections including rectangular, oval, square, triangular, trapezoidal, etc. It is also envisioned that backspan 12 and legs 14 and 16 may have different cross-sectional shapes, e.g., backspan 12 can have a rectangular cross-section and legs 14 and 16 can have an oval cross-section. Legs 14 and 16 can diverge slightly, as shown, although other configurations are envisioned, i.e., legs 14 and 16 can be substantially parallel, converge, etc. The staple may also be configured as a directionally biased staple such as those described in U.S. Patent Application Serial No. 09/972,594, filed November 5, 2001 and incorporated herein in its entirety by reference.

Referring to FIGS. 4-6, the deformed configuration of staple 10 is dependent upon the thickness of the tissue to be fastened. As illustrated in FIG. 4, where the tissue segments "T₁" and "T₂" to be fastened are relatively thin, legs 14 and 16 are deformed against an anvil (not shown), in a known manner, into a modified B-staple configuration wherein the backspan 12 of staple 10 retains or substantially retains its concave configuration. In such a configuration, backspan 12 contacts tissue T₁ to maintain tissue segments T₁ and T₂ in approximation to effect hemostasis. As illustrated in FIG. 5, where the tissue segments T₁ and T₂ are moderately thick, backspan 12 of staple 10 will engage and be partially deformed by tissue segment T₁. Once again, backspan 12 of staple 10 is deformed into a modified B-configuration with backspan 12 contacting tissue segment T₁ to maintain approximation of tissue segments T₁ and T₂ and effect hemostasis. However, backspan 12 may still maintain a generally concave configuration. Finally, as illustrated in FIG. 6, where tissue segments T₁ and T₂ are relatively thick, backspan 12 of staple 10 engages and is deformed by tissue segment T₁ into a substantially B-configuration. Once again, backspan 12 engages tissue segment T₁ to maintain tissue segments T₁ and T₂ in approximation to effect hemostatis. As illustrated in FIGS. 4-6, backspan 12 becomes progressively more linear as the thickness of tissue segments T₁ and T₂ to be joined increases.

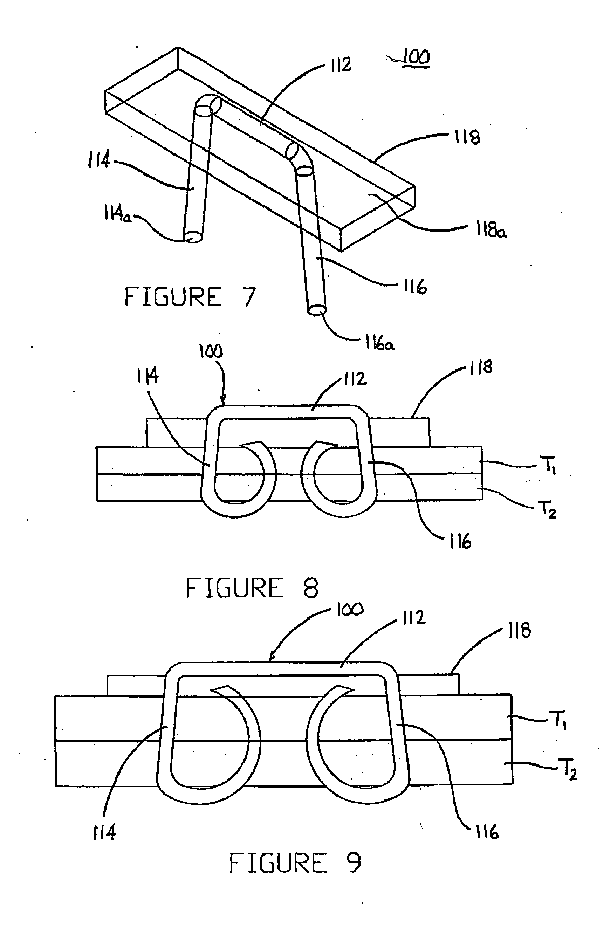

FIG. 7 illustrates an alternate embodiment of the presently disclosed expandable backspan staple shown generally as 100. Staple 100 includes a conventional staple having a backspan 112, a first leg 114, a second spaced leg 116, and a compressible pad or spacer 118. Legs 114 and 116 extend through pad 118 such that a top surface 118a of pad 118 rests against an undersurface of backspan 112. Leg tips 114a and 116a can be tapered to facilitate penetration of tissue as discussed above with respect to staple leg tips 14a and 16a or, in the alternative, be non-tapered or conical in shape. A bottom surface 118b of pad 118 is spaced from top surface 118a in a direction towards tips 114a and 116a.

Pad 118 is formed from a compressible material which may be a polymer, a fluid filled bag, a sponge, or any compressible material suitable for surgical use. It is envisioned that the compressible material can be formed or coated on or about the backspan or attached to the backspan in any known manner.

As illustrated in FIG. 8, when staple 100 is used to fasten relatively thin tissue segments T₁ and T₂, as legs 114 and 116 of staple 100 are passed through tissue segments T₁ and T₂, a bottom surface of pad 118 engages a top surface of tissue segment T₁ to effect and maintain approximation of tissue segments T₁ and T₂ to effect hemostasis. When staple 100 is used to fasten relatively thick tissue segments T₁ and T₂, the bottom surface of pad 118 once again engages a top surface of tissue segment T₁ to effect and maintain approximation of tissue segments T₁ and T₂. However, pad 118 is also be compressed between backspan 112 of staple 100 and tissue T₁ and deformed, i.e., flattened, to accommodate the thicker tissue within staple 100.

The presently disclosed expandable backspan staples may be fitted within cartridges of known surgical stapling instruments including both open and endoscopic instruments and sequential, single, and multiple fire instruments. Examples of such instruments are disclosed in the following U.S. Patents which are incorporated into this application in their entirety by reference: U.S. Patent Nos. 6,045,560, 5,964,394, 5,894,979, 5,878,937, 5,915,616, 5,836,503, 5,865,361, 5,862,972, 5,817,109, 5,797,538 and 5,782,396. It is also envisioned that the presently disclosed embodiments of the expandable backspan staples could also be incorporated into robotically operated surgical staplers.

In another embodiment of the presently disclosed expandable backspan staple, spacer or pad 118 of staple 100 (FIG 7) is supported on staple 10 (FIG. 1). The combined staple (not shown) provides a backspan having two stages of expansion, i.e., the pad will deform first in response to stapling of tissues of moderate thicknesses and the backspan will deform with tissues having greater thicknesses.

It will be understood that various modifications may be made to the embodiments disclosed herein. For example, the above described staple may be formed from any of a variety of surgically acceptable materials including titanium, plastics, resorbable materials, etc. Therefore, the above description should not be construed as limiting, but merely as exemplifications of preferred embodiments. Those skilled in the art will envision other modifications within the scope and spirit of the claims appended hereto.

## Claims

1. A staple comprising:
a non-linear expandable backspan;
a first leg having a first end extending from one end of the expandable backspan and a second end; and
a second leg having a first end extending from the other end of the expandable backspan and a second end;
the non-linear expandable backspan including a central portion extending towards the second ends of the first and second legs and being deformable in a direction away from the second ends of the first and second legs, wherein the backspan is configured to deform upon application to tissues of varying thicknesses, wherein the amount of deformation of the backspan is proportional to the thickness of tissue being stapled.

2. A staple according to Claim 1, wherein the second ends of the First and second legs of the staple are configured to penetrate tissue.

3. A staple according to Claim 1, wherein the backspan has a concave configuration.

4. A staple according to Claim 1, wherein the backspan has a u-shaped configuration.

5. A staple according to Claim 1, wherein the backspan defines a recess.

6. A staple according to Claim 1, wherein the configuration of the recess is selected from the group consisting of u-shaped, trapezoidal and rectangular.

7. A staple according to Claim 1, wherein the backspan has a circular cross-section.

8. A staple according to Claim 7, wherein each of the first and second legs has a circular cross-section.

9. A staple according to Claim 1, wherein the non-linear backspan defines a substantially trapezoidal shape.

10. A staple according to Claim 1, wherein the non-linear backspan defines a substantially rectangular shape.
